# EUROPEAN PATENT APPLICATION

(11) **EP 1 424 388 A1**
(43) Date of publication of application: **02.06.2004**
(21) Application number: 02026462.8
(22) Date of filing: 27.11.2002
(51) Int. Cl.: C12N 5/06

(54) **Method of enriching and/or propagating non-embryonic stem cells while preventing differentiation**

(71) Applicant: MainGen Biotechnologie GmbH, 60314 Frankfurt/Main (DE)
(72) Inventor: Knaus, Rainer, Dr., 63773 Goldbach (DE); Schröder, Bernd, Dr., 17166 Hohen Demzin (DE); Rose-John, Stefan, Dr., 24211 Schellhorn (DE)
(74) Representative: Schüssler, Andrea, Dr.

(57) **Abstract**

The present invention relates to a method of enriching and/or propagating non-embryonic stem cells *ex vivo* such that differentiation of said stem cells is minimized or prevented, wherein said method comprises (a) enriching a source of cells containing stem cells for stem cell specific antigen carrying stem cells, and (b) cultivating the population of cells obtained in step (a) in a serum- and stroma-free medium in the presence of cytokines. The method of the present invention is particularly useful for expanding haematopoietic stem cells *ex vivo*.

## Description

The present invention relates to a method of enriching and/or propagating non-embryonic stem cells ex vivo such that differentiation of said stem cells is minimized or prevented, wherein said method comprises (a) enriching a source of cells containing stem cells for stem cell specific antigen carrying stem cells, and (b) cultivating the population of cells obtained in step (a) in a serum- and stroma-free medium in the presence of cytokines. Preferably, stem cell specific antigen carrying cells are selected by immunomagnetic separation. The method of the present invention is particularly useful for expanding haematopoietic stem cells *ex vivo*.

Embryonic stem (ES) cells are the archetypical stem cells, being capable of differentiating to form the whole gamut of cell types found in the adult animal. Such stem cells are described as pluripotent cells as they are capable of differentiating into many cell types. Although ES cells have been isolated from humans, their use in research as well as therapeutics is encumbered by ethical considerations. Stem cells also exist for most tissues including haematopoietic, neural, gastrointestinal, epidermal, hepatic and mesenchymal stem cells. However, these stem cells have less self-renewal ability and a more restricted capacity for differentiation, i.e. they are not pluripotent.

Until recently, it was thought that tissue-specific stem cells could only differentiate into cells of the tissue of origin. However, recent studies suggested that tissue-specific stem cells can differentiate into lineages other than the tissue of origin. After transplantation of bone marrow or enriched heamatopoietic stem cells, skeletal myoblasts, cardiac myoblasts, endothelium, hepatic and biliary duct epithelium, lung, gut and skin epithelia, and neuroectodermal cells of donor origin have been detected. Some studies demonstrated that neural stem cells as well as muscle cells may differentiate into haematopoietic cells. When injected into a blastocyst, neural stem cells contribute to a number of tissues of the chimeric mouse embryo. However, although there has developed a pressing need to obtain non-embryonic (human) pluripotent stem cells, hitherto the problems associated with producing cultures of such stem cells including the problem of inhibiting (further) differentiation have not been solved.

Thus, the technical problem underlying the present invention is to provide means allowing to expand *ex vivo* non-embryonic stem cells so as to avoid substantial differentiation.

The solution to the above technical problem has been achieved by providing the embodiments characterized in the claims. It has surprisingly been found that haematopoietic stem cells can be expanded *ex vivo* without substantial differentiation by, first, enriching the source used, e.g. bone marrow, peripheral blood or cord blood, for haematopoietic stem cells. By using immunomagnetic separation, CD34⁺ cells cells could be enriched so as to constitute more than 80% of the cell population. In the second step, the cell population enriched for CD34+ cells was cultivated for 7 days by use of a closed culturing system in a serum- and stroma-free medium in the presence of the cytokines SCF, TPO and Ftl-3 with or without Hyper-IL-6. By use of this method, an impressive expansion of total number of cells, colony forming cells (CFU), CD34⁺ cells and late haematopoietic stem cells could be achieved and a slight expansion of early stem cells while preventing substantial further differentiation.

### Brief description of the drawings

Figure 1A: Percentages of expansion of various cell types after cultivation for 7 days as described in Example 1
Figure 1B: Enhanced expansion of CD34+ cells from blood after stimulation with Hyper-IL-6
Figure 2: Dependence of the percentages of expansion of CFU and LTC-IC from the purity of the starting cell population
Figure 3: Rate of expansion of CD34⁺ cells from cord blood (CB) at various cell concentrations [5x10³ to 2x10⁵ cells/ml volume]
Figure 4: Comparison of the rates of expansion in the presence or absence of IL-3
Figure 5: Percentage of human CD45⁺ cells in bone marrow of NODSCID mice (cells were expanded for 7 days)
Figure 6: Decrease of the amount of CD34⁺ cells during culturing
Figure 7: Expansion of CD34⁺ cells within 14 days

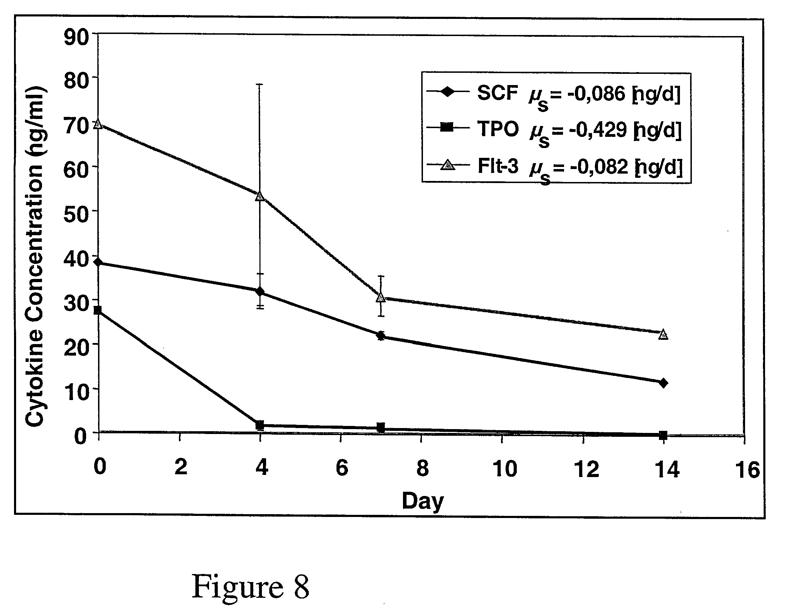

Figure 8: Cytokine consumption rate within 14 days expansion of CD34+ cells

The present invention relates to a method of enriching and/or propagating non-embryonic stem cells ex vivo such that differentiation of said stem cells is minimized or prevented, wherein said method comprises the following steps:
(a) Enriching a source of cells containing stem cells for stem cell specific antigen carrying stem cells; and
(b) cultivating the population of cells obtained in step (a) in a serum- and stroma-free medium in the presence of at least three cytokines for a sufficient period of time wherein said cytokines comprise TPO, SCF, Flt-3-lig, PDGF, EGF, IL-3, VEGF or Hyper-IL-6.

As used herein, the term "non-embryonic stem cells" comprises any non-embryonic stem cells, e.g. a gonadal stem cells, somatic stem/progenitor cells, haematopoietic stem cells, epidermal stem cells or neuronal stem cells with haematopoietic stem cells being preferred. Sources for haematopoietic stem cells are, e.g., cord blood (CB), bone marrow or peripheral blood. Sources for the further stem cells listed above are, e.g., bone marrow, cord blood, peripheral blood, fat tissue, liver, muscle, skeletal myoblasts, cardiac myoblasts, endothelium and epithelium.

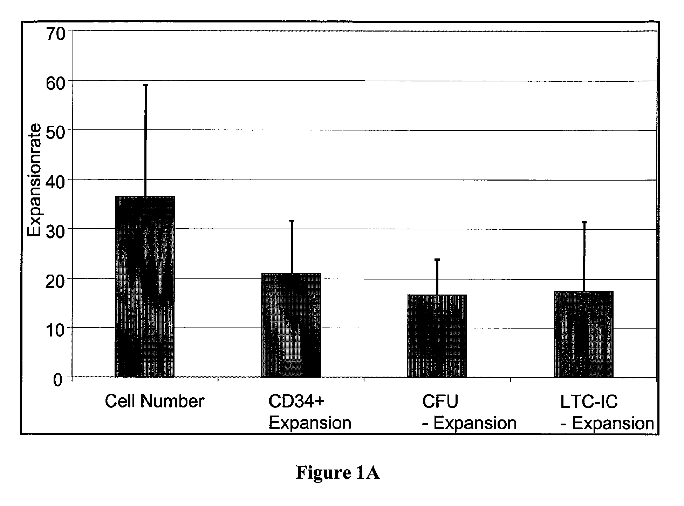

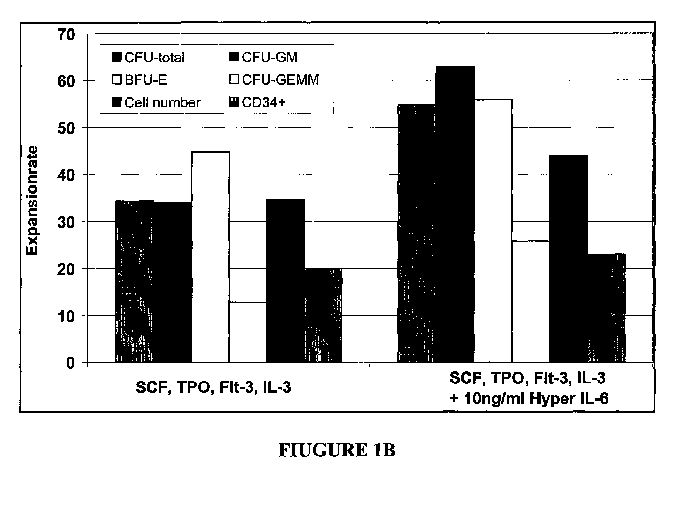

As used herein, the term "minimizing or preventing differentiation" refers to culturing conditions wherein the stem cells substantially maintain the status of potency which they had before expansion according to the method of the present invention. Additionally, in the method of the present invention the total number of late stem cells, different types of progenitor cells and the total number of cells could be expanded; see Figure 1.

The person skilled in the art knows suitable methods for culturing stem cells and suitable media. As used herein, the term "serum- and stroma-free medium" means any serum- and stroma-free medium which is generally used for cultivation of mammalian (human) cells and suitable serum- and strom-free media are commercially available. In a preferred embodiment, the basal serum- and stroma-free medium is cellgro (Cellgenix, Freiburg, Germany) or X-vivo 10 (BioWittaker, Verviers, Belgium). For step (b), the initial cell density should be at least 5x10³ cells per ml medium but should not exceed 2x10⁵ cell per ml medium. Preferably, the expansion of the cells (step b)) is carried out at 37°C in a humidified 95% air/5% CO₂ atmosphere for at least 7 days. Preferably, during this period of time the culture is not agitated and is not manipulated in any way, e.g., by taking samples or replacing the medium.

Stem cell specific antigens are known to the person skilled in the art and comprise, e.g., CD34, AC133, c.Kit, Thy-1, KDR (VEGF-Receptor), Stro-1 or, in the opposite, the depletion of cells which express lineage marker (Lin- cells).

Preferred concentrations of cytokines in the culture medium for expansion are 6 to 60 ng/ml TPO, 15 to 150 ng/ml SCF and 15 to 150 ng/ml Flt-3-lig, with concentrations of about 20 ng/ml (TPO), 50 ng/ml (SCF) and 50 ng/ml (Flt-3-lig) being particularly preferred.

In a preferred embodiment of the method of the present invention, enrichment of stem cell secific antigen carrying stem cells is carried by immunoisolation using suitable antibodies or fragments thereof. Techniques for immunoisolation are well known to the person skilled in the art. A preferred method of immunoisolation is immunomagnetic separation as, e.g., described in Example 1, below.

In a more preferred embodiment of the method of the present invention, step (a) is conducted in such a way that after completion at least 80% of cells of the cell population are stem cell specific antigen carrying stem cells. This can be achieved, e.g., by using immunomagnetic separation as described in Example 1, below.

In an even more preferred embodiment of the method of the present invention, for step (b) at least one additional cytokine is added, preferably at day 0 of step (b). Particularly useful are cytokines like IL-3 or an IL-6/IL-6R-fusion protein. An example of such a fusion protein is Hyper-IL-6 (IL-6/sIL-6R fusion polypeptide), i.e., a fusion polypeptide comprising a human sIL-6R polypeptide and a human IL-6 polypeptide, wherein both polypeptides are linked by a polypeptide linker. The structure of Hyper-IL-6 as well as its amino acid sequence are described in DE 196 08 813 C2 and in Fischer et al., Nature Biotechnology 15 (1997), 142-145. As used herein, the term "Hyper-IL-6" comprises (a) the fusion polypeptide having the amino acid sequence disclosed in DE 196 08 813 C2 and (b) a fusion protein having an amino acid sequence differing from the amino acid sequence of (a) with substantially the same biological activity. The preferred concentrations of the additional cytokines are in the range of 3 to 30 ng/ml medium with concentrations of about 10 ng/ml being particularly preferred (see also Example 1b).

Any cultureware can be used for the method of the present invention, however, plastics bags are preferred for cultivation. Preferably, said plastic bag consists of a perfluorated polymer.

In a further preferred embodiment of the method of the present invention, in step (b) cultivation is carried out in the presence of (an) inhibitor(s), preferably an antibody, directed against inhibiting and/or differentiation inducing compounds. Preferably, said inhibitor is an anti-TGF-β- or an anti-TNF-α-antibody.

The following Examples illustrate the invention.

### Example 1: Expansion of human haematopoietic stem cells

### (A)

Umbilical cord blood was collected immediately after delivery in a sterile tube containing 5000 I.E. Heparin. Informed consent of the mother was obtained. Mononuclear cells (MNC) were isolated prior to processing of the cord blood sample over Ficoll/Hypaque density cushion. The CD34+ selection was performed with the Miltenyi MiniMACS column according to the manufacturer's instruction. Briefly, UCB MNC were incubated for 15 min at 4°C with human IgG to block the Fc receptors and an anti-CD34 antibody (QBEND10, Miltenyi Biotec, Bergisch Gladbach, Germany) modified with a hapten. Cells were washed with PBS containing 0.5% bovine serum albumin (BSA) (Sigma, Germany) and 0.1% EDTA (Titriplex III, Merck, Darmstadt, Germany) (referred to as MACS buffer) and incubated for 15 min at 4°C with an anti-hapten mouse monoclonal antibody conjugated to colloidal superparamagnetic beads (Miltenyi Biotec). Labelled cells were applied to magnetic column (MS column, Miltenyi Biotec), unbound cells washed out and CD34+ cells eluted from the column with MACS buffer. To improve the purity of the CD34+ cells a second purification cycle was performed using a smaller column (VS column, Miltenyi Biotec).

The final cell population obtained having a purity of about 84-94% (CD34+) was used as starting cell population for the propagation of the stem- and progenitor cells *ex vivo*.

The cell population was seeded in culture plates (concentration: 5x10⁴ cells per ml culture medium) and cultivated. The culture medium used was X-Vivo 10 (BioWittaker) which had been supplemented with the following cytokines: Interleukin 3 (IL-3) (10 ng/ml); stem cell factor (SCF) (50 ng/ml); Flt-3-ligand (Flt-3-lig) (50 ng/ml) and Thromopoietin (TPO) (20 ng/ml) (R&D, Wiesbaden, Germany). Cells were cultivated for 7 days without replacement of medium. At day 7, cells were harvested and the following parameters were evaluated: (1) number of cells, (2) percentage of CD34⁺ cells, (3) percentage of "colony forming units" (CFU) (corresponding to the percentage of progenitor cells); and (4) percentage of the "long-term culture initiating cells" (LTC-IC) (corresponding to the percentage of stem cells).

The results are shown in Figure 1 demonstrating the remarkable expansion of early and late progenitor cells, late stem cells, CD34+ cells and cells in general.

### (B)

After informed consent of the mother was obtained, placental blood was collected from the unborn placenta. The collected Cord Blood was transported to the Red Cross Cord Blood Bank, Mannheim Germany. The units were processed and cryopreserved within 48 hours after blood collection. For the experiments cryopreserved Cord Blood (Red Cross Cord Blood Bank, Mannheim Germany) was used.

The Cord Blood samples were thawed according to a modified technique described by Rubinstein et al., Proc. Natl. Acad. Sci. USA 1995;92:10119-10122. The samples were thawed quickly within a water bath at 37°C, and diluted in an equal volume of washing solution (12.5% (vol/vol) HSA 20% (BSD, Frankfurt, Germany) plus 50% (vol/vol) Dextran 40 within saline solution (Fresenius, Bad Homburg, Germany). After centrifugation, the supernatant was removed, and the cell pellet was resuspended in washing solution (PBS, 1% NaCitrat Baxter, Unterschleißheim, Germany, 1% HSA). The CD34+ cells were enriched by the medical device "CliniMACS" system (Miltenyi, Bergisch Gladbach, Germany). The whole production process was performed under GMP compliant.

The enriched CD34+ cells were seeded in a cell concentration of 5x10⁴ cells per well in 12 well plates (Nunc, Wiesbaden, Germany) and incubated in an incubater at 37°C and 95% humidity. The expanded cell population was collected after day 7 and the total cell number, portion of CD34+ cells and different kinds of CFU were analysed. The results are shown in Figure 1b. Due to the addition of Hyper-IL-6 to the standard cytokine cocktail all kinds of cell population could be enhanced 2 fold.

### Example 2: Influence of the purity of the fraction of CD34⁺ cells on expansion of CFU and LTC-IC

In this example, the influence of purity on the capability of the cells to propagate was studied. CD34⁺ cells were cultivated for 7 days under the same conditions a described in Example 1.

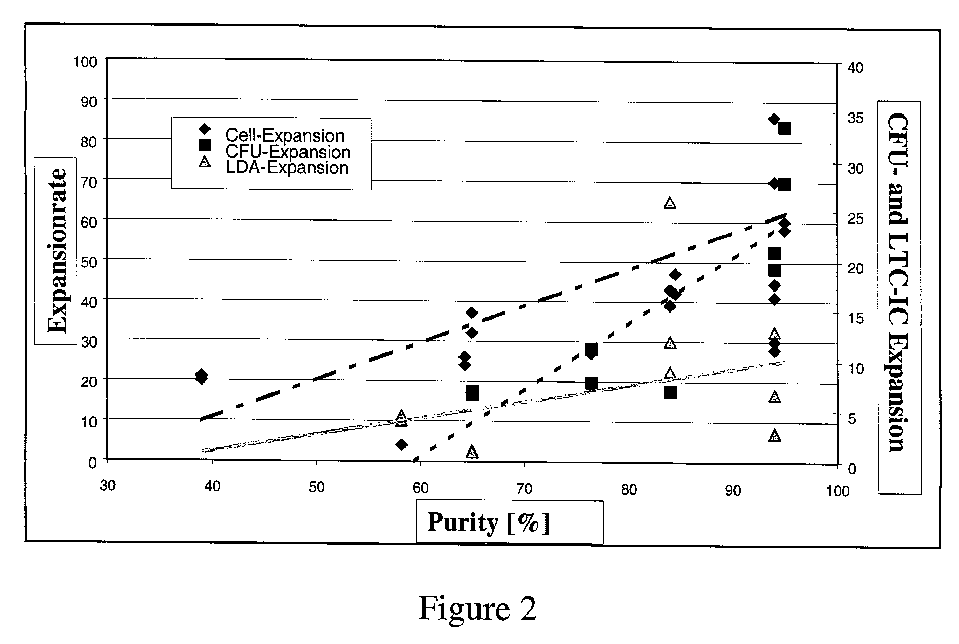

The results are summarized in Figure 2. As shown in Figure 2, when using CD34⁺ cell populations having a higher purity for further cultivation, within 7 days the expansion of the total number of cells, the colony forming cells (CFU) and the LTC-IC is clearly enhanced (N=7). It can be inferred from the figure that only when using a purity higher than 80% an expansion of stem cells (LTC-IC) of more than eight-fold (which is required for grafting) can be achieved.

### Example 3: Influence of the cell concentration on expansion of the total number of cells, CD34⁺ cells and CFU

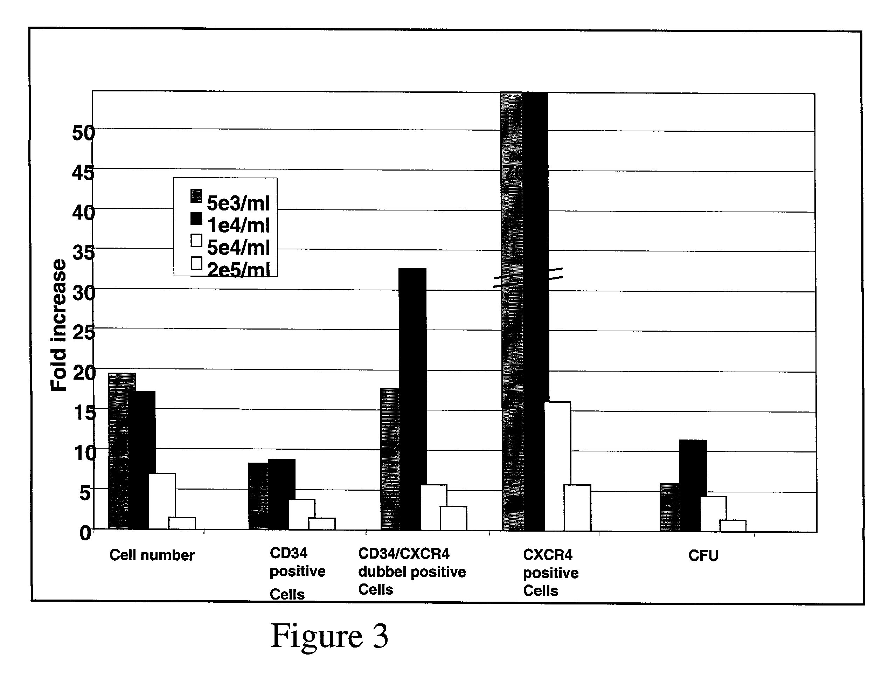

In this example, the influence of the concentration of cells on the capability of stem cells to propagate was investigated. Again, CD34⁺ cells were cultivated for 7 days under the same conditions a described in Example 1. In Figure 3 the rates of expansion at cell concentrations between 5x10³ and 2x10⁵ cells per ml medium are shown. An optimum rate of expansion of the total number of cells, CD34⁺ cells and CFU can be found between 5x10³ and 5x10⁴ cells per ml medium. Accordingly, for optimum expansion the concentration of cells at the beginning of culturing for expansion should be within that range.

### Example 4: Influence of combinations of various cytokines on expansion of stem cells

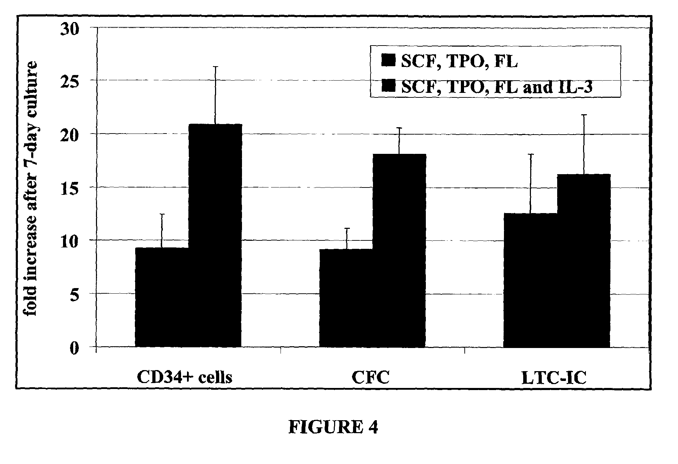

The influence of the presence of IL-3 in combination with TPO, SCF and Flt-3-lig was studied. The cytokines had the following concentrations: IL-3, 10 ng/ml; TPO, 20 ng/ml; SCF, 50 ng/ml; Ftl-3-lig, 50 ng/ml. For studying the effects of these cytokines, the increase of the total number of cells, CFU, CD34⁺-antigen carrying cells and stem cells (LTC-IC) was determined. The results are shown in Figure 4. Figure 4 demonstrates that by use of a combination of cytokines SCF, Flt-3-lig and TPO a consistently good propagation of all types of cells can be achieved. This effect can be further enhanced by addition of IL-3.

### Example 5: Engraftment of expanded cells in bone marrow of NODSCID mice

Cells expanded as described in Example 4 were investigated as regards their capability to engraft in bone marrow of NODSCID mice. The reconstitution of NOD/SCID mice with human hematopoietic cell aliquots consisting of 1 x 10⁵ cord blood CD34+ cells and the corresponding progeny generated after ex vivo expansion which were transplanted into NOD/SCID mice aged 4 to 8 weeks. Nonobese diabetic/severe combined immunodeficient (NOD/SCID) breeding pairs were obtained from Jackson Laboratories (Pearl Harbour, Maine, USA). The mice were bred and maintained in the animal facility of the ASTA MEDICA (Frankfurt, Germany). Prior to transplantation of the cord blood cells, the mice were sublethally irradiated with 100 cGy and were cotransplanted with irradiated rat fibroblasts in Matrigel genetically modified to produce human IL-3. Six weeks after transplantation the mice were sacrificed. Both femurs were prepared and the presence of the human hematopoietic cells in the bone marrow of the NOD/SCID recipients was analysed by flow cytometry. Aliquots of the murine BM cells were incubated with antibodies directed against the human common leukocyte antigen CD45 or double stained with antihuman CD45-FITC in combination with antihuman CD34-PE for 20 min at 4°C. For analysis of multilineage engraftment the cells were incubated with antihuman CD33-PE and antihuman CD19-FITC (both Becton Dickinson, Heidelberg, Germany). Thereafter, red cells were lysed by adding 2 ml FACS lysing solution (Becton Dickinson, Heidelberg, Germany) and incubated for 30 min at room temperature. Cells were then washed in FACS wash (Becton Dickinson, Heidelberg, Germany), resuspended and analysed by flow cytometry. 10.000 to 50.000 events were counted.

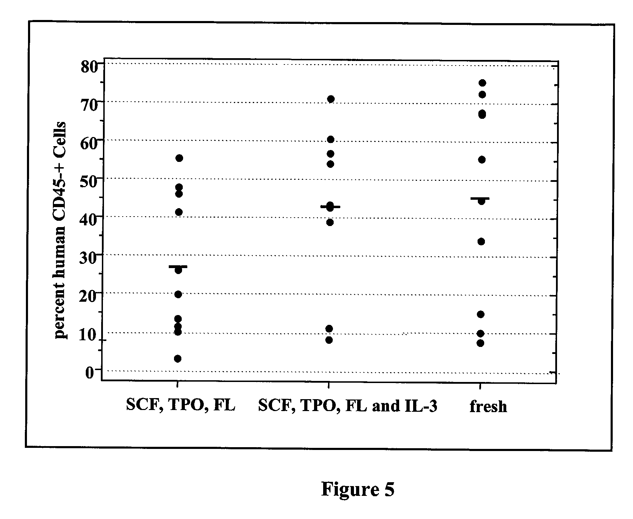

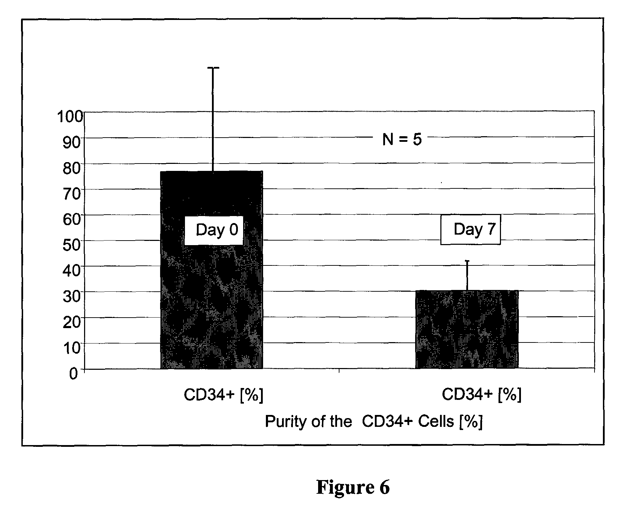

In Figure 5, the engraftment of expanded stem cells in bone marrow of immuno-incompetent mice is shown as percentage of human cells in bone marrow. The summarized results of both independently carried experiments clearly show that by expanding in the presence of the cytokines SCF, TPO, Flt-3-lig and IL-3 for 7 days the percentage of human cells in bone marrow of NODDSCID mice corresponds to the percentage of non-expanded cells. Thus, the percentage of stem cells and, therefore, the stem cell potential of the graft should correspond to the percentage of non-expanded cells. Moreover, it could be shown that within the first seven days of culturing the percentage of the total number of cells could be increased by a factor of 38 and the percentage of progenitor cells (CD34⁺, CFU) and late stem cells by a factor 18 allowing a clearly accelerated establishment of the haematopoietic system after grafting. During cultivation, the percentage of CD34⁺ cells decreases from more than 80% to about 30%; see Figure 6.

### Example 6: Rates of expansion of different cell types within a cultivation period of 14 days

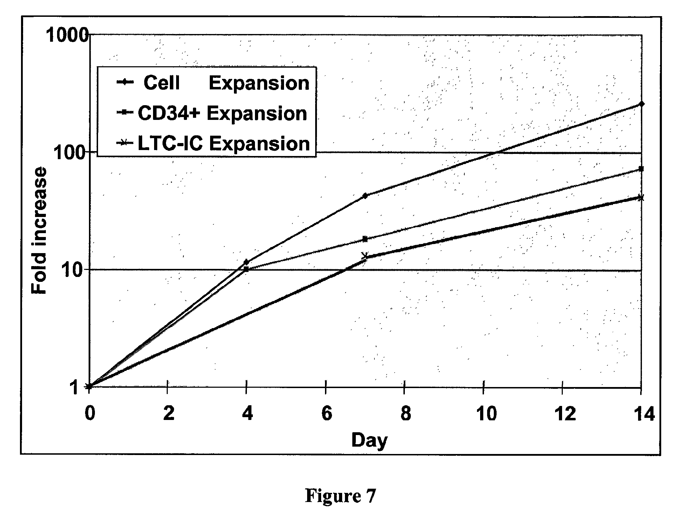

Cells were cultivated for 14 days as described in the previous examples and the rates of expansion of various types of cells are shown in Figure 7. The figure shows that all types of cells (total number of cells, Cd34⁺ cells, LTC-IC) can be propagated within a cultivation period of 14 days.

### Example 7: Influence of cytokine concentration on expansion of stem cells

In this example, the concentration of the cytokines TPO, SCF and Flt-3-lig during the course of cultivation for expansion was determined in the culture medium. As shown in Figure 7, at day 4 the initial concentration of TPO (20 ng/ml) drastically decreases and, thus, it can be concluded that at day 4 TPO does no longer show any biological effect. Similarly, at day 4 IL-3 can no longer be detected (data not shown). Thus, the concentrations of cytokines used in Example 7 (IL-3, 10 ng/ml; TPO, 20 ng/ml; SCF, 50 ng/ml; Flt-3-lig, 50 ng/ml) are advantageous, since, on the one hand, at the beginning of culturing IL-3 strongly stimulates propagation of all cells (however, at day 4 is no longer present), on the other hand, at the beginning of culturing TPO inhibits adoptosis and, due its decreasing concentration, after day 4 only the stem cell factors SCF and Flt-3-lig stimulate the growth of CD34⁺ cells while preventing differentiation of the stem cells. Accordingly, the combination of cytokines and the concentrations used in the present example can be regarded as constituting optimum conditions for expansion of highly purified CD34⁺ cells.

## Claims

1. Method of enriching and/or propagating non-embryonic stem cells ex vivo such that differentiation of said stem cells is minimized or prevented, wherein said method comprises the following steps:
(a) Enriching a source of cells containing stem cells for stem cell specific antigen carrying stem cells; and
(b) cultivating the population of cells obtained in step (a) in a serum- and stroma-free medium in the presence of at least three cytokines for a sufficient period of time wherein said cytokines comprise TPO, SCF, Flt-3-lig, PDGF, EGF, IL-3, VEGF or Hyper-IL-6.

2. The method of claim 1, wherein said stem cell specific antigen carrying stem cells are enriched by immunoisolation.

3. The method of claim 2, wherein said immunoisolation is immunomagnetic separation.

4. The method of any one of claims 1 to 3, wherein after step (a) at least 80% of cells are stem cell specific antigen carrying stem cells.

5. The method of any one of claims 1 to 4, wherein in step (b) the cells are cultivated for at least 7 days without replacing the culture medium.

6. The method of any one of claims 1 to 5, wherein said non-embryonic stem cells are haematopoietic stem cells and said source of stem cells is bone marrow, peripheral blood or cord blood.

7. The method according to any one of claims 1 to 6, wherein for step (b) at least one additional cytokine is added.

8. The method of claim 7, wherein said additional cytokine is IL-3 or an IL-6/IL-6R-fusionprotein.

9. The method of any one of claims 1 to 8, wherein a plastic bag is used for cultivation.

10. The method of claim 9, wherein said plastic bag consists of a perfluorated polymer.

11. The method of any one of claims 1 to 10, wherein said stem cell specific antigen is CD34, Ac133, c-Kit or Thy-1.

12. The method according to any one of claims 1 to 11, wherein in step (b) cultivation is carried out in the presence of (an) inhibitor(s) directed against inhibiting and/or differentiation inducing compounds.

13. The method of claim 12, wherein said inhibitor is an anti-TGF-β- or an anti-TNF-α-antibody.
